# EUROPEAN PATENT SPECIFICATION

(11) **EP 2 656 078 B1**
(45) Date of publication and mention of the grant of the patent: **26.10.2016**
(21) Application number: 11819026.3
(22) Date of filing: 21.12.2011
(51) Int. Cl.: G01N 33/68, G01N 33/574

(54) **A METHOD OF DIFFERENTIATING ONCOGENIC CHANGES OF THE THYROID, A KIT TO EMBODY THE METHOD AND THE USE OF METALLOTHIONEIN (MT) FOR DIFFERENTIATING ONCOGENIC CHANGES OF THE THYROID**
VERFAHREN ZUR DIFFERENZIERUNG ONKOGENER VERÄNDERUNGEN DER SCHILDDRÜSE, KIT ZUR UMSETZUNG DES VERFAHRENS UND VERWENDUNG VON METALLTHIONEIN (MT) ZUR UNTERSCHEIDUNG VON ONKOGENEN VERÄNDERUNGEN DER SCHILDDRÜSE
PROCÉDÉ VISANT À DIFFÉRENCIER DES MODIFICATIONS ONCOGÉNIQUES DE LA THYROÏDE, KIT POUR RÉALISER LE PROCÉDÉ ET UTILISATION DE LA MÉTALLOTHIONÉINE (MT) POUR DIFFÉRENCIER DES MODIFICATIONS ONCOGÉNIQUES DE LA THYROÏDE

(30) Priority: 22.12.2010 PL 39338910
(43) Date of publication of application: 30.10.2013
(73) Proprietor: Wroclawskie Centrum Badan EIT + Sp. z o.o., 54-066 Wroclaw (PL)
(72) Inventor: DZIEGIEL, Piotr, PL-51-650 Wroclaw (PL); PODHORSKA-OKOLÓW, Marzena, PL-53-303 Wroclaw (PL); KRÓLICKA, Anna, PL-51-153 Wroclaw (PL); KOBIERZYCKI, Christopher, PL-51-144 Wroclaw (PL); PULA, Bartosz, PL-52-210 Wroclaw (PL); PIOTROWSKA, Aleksandra, PL-54-104 Wroclaw (PL)
(74) Representative: Witek, Rafal
(86) International application number: PCT/PL2011/050052
(87) International publication number: WO 2012/087170

(56) References cited:
- WO-A2-2007/093177
- JP-A- 2011 088 866
- SCHMID K W ET AL: "Metallothionein expression in normal, hyperplastic, and neoplastic thyroid follicular and parafollicular c cells using monoclonal antimetallothionein antibody E9", ENDOCRINE PATHOLOGY, HUMANA PRESS, TOTOWA, NJ, US, vol. 5, no. 2, 1 January 1994 (1994-01-01) , pages 114-122, XP009157893, ISSN: 1046-3976, DOI: 10.1007/BF02921379
- ELMES M E ET AL: "DIAGNOSTIC APPLICATIONS OF METALLOTHIONEIN IMMUNOCYTOCHEMISTRY IN MAN", JOURNAL OF TRACE ELEMENTS IN EXPERIMENTAL MEDICINE, WILEY-LISS, US, vol. 5, no. 2, 1 January 1992 (1992-01-01) , page 79, XP009157895, ISSN: 0896-548X
- REMMELE W ET AL: "Vorschlag zur einheitlichen Definition eines Immunreaktiven Score (IRS) für den immunhistochemischen Östrogonrezeptor-Nachweis (ER-ICA) im Mammakarzinomgewebe = Recommendation for uniform definition of an immunoreactive score (IRS) for immunohistochemical estrogen receptor detection (ER-ICA) in brea", PATHOLOGE, BERLIN, DE, vol. 8, no. 3, 1 May 1987 (1987-05-01), pages 138-140, XP009129710, ISSN: 0172-8113
- NARTEY N ET AL: "Immunohistochemical localization of metallothionein in human thyroid tumors", AMERICAN JOURNAL OF PATHOLOGY; [10640], AMERICAN SOCIETY FOR INVESTIGATIVE PATHOLOGY, US, vol. 129, no. 1, 1 October 1987 (1987-10-01), pages 177-182, XP009157901, ISSN: 0002-9440
- LIU Z M ET AL: "Expression of functional metallothionein isoforms in papillary thyroid cancer", MOLECULAR AND CELLULAR ENDOCRINOLOGY, ELSEVIER IRELAND LTD, IE, vol. 302, no. 1, 10 April 2009 (2009-04-10), pages 92-98, XP025988968, ISSN: 0303-7207, DOI: 10.1016/J.MCE.2008.12.017 [retrieved on 2009-01-20] cited in the application
- SHIMA T ET AL: "Modification of dietary copper levels on the early stage of tumor-promotion with propylthiouracil in a rat two-stage thyroid carcinogenesis model", CHEMICO-BIOLOGICAL INTERACTIONS, ELSEVIER SCIENCE IRLAND, IR, vol. 180, no. 2, 15 July 2009 (2009-07-15) , pages 262-270, XP026161865, ISSN: 0009-2797, DOI: 10.1016/J.CBI.2009.02.002 [retrieved on 2009-02-13]
- FERRARIO CRISTINA ET AL: "Metallothionein 1G acts as an oncosupressor in papillary thyroid carcinoma", LABORATORY INVESTIGATION, vol. 88, no. 5, May 2008 (2008-05), pages 474-481, XP002672634, ISSN: 0023-6837 cited in the application
- PEDERSEN M O ET AL: "The role of metallothionein in oncogenesis and cancer prognosis", PROGRESS IN HISTOCHEMISTRY AND CYTOCHEMISTRY, GUSTAV FISCHER VERLAG, STUTTGART, DE, vol. 44, no. 1, 22 April 2009 (2009-04-22) , pages 29-64, XP026064696, ISSN: 0079-6336, DOI: 10.1016/J.PROGHI.2008.10.001 [retrieved on 2008-12-01]
- KRÓLICKA ANNA ET AL: "Comparison of metallothionein (MT) and Ki-67 antigen expression in benign and malignant thyroid tumours", ANTICANCER RESEARCH GREECE,, vol. 30, no. 12, 1 December 2010 (2010-12-01), pages 4945-4949, XP009157896, ISSN: 1791-7530

## Description

The subject of the present invention is a method of differentiating oncogenic changes of the thyroid encompassing follicular adenoma and follicular cancer, and the use of metallothionein for differentiating the abovementioned types of oncogenic changes of the thyroid. The subject of the present invention is in the area of diagnosis and of differentiating tumours of thyroid using immunohistochemical methods.

Thyroid cancers are the most frequent tumours of the endocrine system. Cells of the thyroid follicles develop into highly differentiated cancers (papillary and follicular), which occur most frequently in young persons, and poorly differentiated anaplastic cancers, which usually occur in persons over 60 years of age. Papillary cancer constitutes 70-80% of thyroid tumours and is perceived as the mildest of the cancers of this organ. Follicular cancer in turn constitutes 5-15% of thyroid cancers. Due to the difficulty of differentiating it from the follicular adenoma, which is a mild change, both of them are termed a follicular tumour. Their differentiation is possible only using postoperative isolates, but are not differentiable using thin needle biopsies.

One of the most commonly diagnosed proliferation markers is Ki-67. Elevated Ki-67 expression has been shown, amongst others, in cancers of the breast, lung, prostate, soft tissue and brain tumours, but its biological role has not yet been discerned. In many reports published so far, the expression of metallothionein (MT) and antigen Ki-67 was compared in various types of tumours. To date, studies were performed on tumours of the colon, adrenal cortex, oral cavity, prostate, mammary glands, ovary, soft tissue sarcomas and non-small cell lung cancer. In most cases a clear correlation was observed between MT and Ki-67 antigen expression. Nevertheless, research to date on the expression of MT and its isoforms in benign and malignant changes of the thyroid has often yielded confusing results.

Prior art includes the publication by Ferrario et al. (Ferrario C, Lavagni P, Gariboldi M, Miranda C, Losa M, Cleris L, Formelli F, Pilotti S, Pierotti MA and Greco A: Metallothionein 1G acts as an oncosupressor in papillary thyroid carcinoma. Lab Invest 88: 474-481, 2008), in which the authors demonstrate a decrease in the expression of functional isoforms of MT in papillary cancer (MT1E, MT1G, MT1F and MT2A) and demonstrated in a cell model that MT1G plays a possible role of an oncogenic process suppressor. The publication solely related to the question of differentiating malignant type changes in papillary cancer and follicular cancer, whereas did not touch at all upon the diagnosis problem of follicular adenoma and follicular cancer. The Huang Y. report revealed that the decreased MT-1G expression in thyroid papillary cancer occurs as a result of the hypermethylation of its promoter (Huang Y, de la Chapelle A and PelIegata NS: Hypermethylation, but not LOH, is associated with the low expression of MT1G and CRABPI in papillary thyroid carcinoma. Int J Cancer 104: 735-744, 2003). Huang Y. shows results of studies on healthy thyroid tissue and papillary cancer and its follicular variant (FVPTC). However, no observations are made on the follicular adenoma and the diagnostic possibilities of this type vs. follicular cancer. Liu et al. demonstrate that each of the 8 functional isoforms of MT may be expressed as a result of the effect of the stimulation by cadmium and the activation of ERK1/2 kinases in cells of thyroid papillary cancer. Cells, in which these isoforms have been expressed are characterised by a shorter G1 phase and a more rapid transition into the S and M phases of the cell cycle (Liu ZM, Hasselt CA, Song FZ, Vlantis AC, Cherian MG, Koropatnick J and Chen GG: Expression of functional metallothionein isoforms in papillary thyroid cancer. Mol CelI Endocrinol 302 :92-98, 2009). The work of Liu et al. on the expression of metallothionein in thyroid tumours was performed only on one line of papillary cancer (KAT5) isolated from material from one patient. The report fails to supply research results on clinical material from different patients, which would assure a variety of cases and illustrate the factual state. The authors of the publication also fail to attend to the diagnostic problem of follicular adenoma in relation to follicular cancer and concentrate on an analysis of the mechanisms regulating the level of expression of MT in a papillary cancer line.

MT isoform induction also occurs in cells of anaplastic thyroid cancer effected by calcium ions, wherein their increased expression also resulted in a shortening of the G1 phase of the cycle (Liu ZM, Chen GG, Shum CK, Vlantis AC, Cherian MG, Koropatnick J and van Hasselt CA: Induction of functional MT1 and MT2 isoforms by calcium in anaplastic thyroid carcinoma cells. FEBS Lett. 581:2465-2472, 2007). Research performed on this also do not touch upon the possibility of differentiating follicular cancer from follicular adenoma using MT expression measurements.

There is thus a great need to deliver an effective marker of oncogenic changes of the thyroid of the follicular adenoma and follicular cancer types, which would facilitate the correct diagnosis and selection of the correct therapeutic intervention. Despite many years' research on the role of the metallothionein protein in various malignant tumours its high prognostic and predictive value has not been confirmed for said types of thyroid tumours.

The subject of the present invention is a method of differentiating oncogenic changes of the thyroid encompassing follicular adenoma and follicular cancer *in vitro,* characterised in that an oncogenic tissue sample from a patient is examined for the expression of the metallothionein (MT) protein using an immunohistochemical method using anti-MT monoclonal antibodies, the degree of increased expression of metallothionein is evaluated under a light microscope using the semiquantitative IRS method according to Remmele, wherein the degree of increase in the expression of metallothionein is equal to or greater than 8 on the semiquantitative IRS scale, and therefore the presence of follicular cancer is ascertained.

Preferentially, the expression of the MT I and MT II isoforms of metallothionein is evaluated

Preferentially, metallothionein expression is examined in material from patients after thyrectomy and tumour removal.In another preferable embodiment of the present invention, the presence of follicular cancer or follicular adenoma is determined with a simultaneous histopathological examination for the abovementioned changes.

The next subject is a kit for differentiating oncogenic changes of the thyroid encompassing follicular adenoma and follicular cancer containing anti-MT monoclonal antibodies.

In a preferred embodiment, the kit contains monoclonal antibodies directed against the MT I and II isoforms of metallothionein.

Another subject of the present invention is the use of metallothionein for differentiating thyroid tumours of the follicular cancer and follicular adenoma types.

Preferentially, the isoforms of metallothionein are MT I and II.

The metallothioneins (MT) are a family of proteins of low molecular mass, whose presence has mainly been noted in animals. They are highly conserved and occur in the form of 4 main isoforms in various locations of healthy and oncogenic tissue. The MT polypeptide chain is composed of from 61 to 68 amino-acids (depending on the isoform) in which 30% of amino-acids residues are cysteine residues. An MT molecule is composed of two domains connected by a lysine dimer. The physiological role of MT is to bind and transport zinc and copper ions and detoxify the organism (by binding) heavy metals, i.e. cadmium, mercury and lead, in intensively dividing cells (including oncogenic cells) it was observed that the expression of MT increases which delivers zinc ions (Zn) to enzymes participating in DNA replication, as well as the increased expression of MT protecting cells against apoptosis. MTs may also influence the activity of a number of chemotherapeutics (they deactivate the free radicals formed during the metabolism of some cytostatics such as anthracyclins, and may bind with others, such as cisplatin). Differences in the expression of MT between follicular adenoma and follicular cancer indicate the possibility of using MT as an ancillary marker in the differentiation of the abovementioned changes. MT is also a much better diagnostic marker differentiating between changes of the follicular adenoma and follicular cancer types than Ki-67, because there is a statistically significant difference between these types of tumours in the expression of MT. There is a lack of significant differences in the expression of antigen Ki-67. The use of the subject of the diagnostic methods constitutes a supplemental method for the difficult histopathological diagnostics of these two hypertrophic changes of the thyroid.

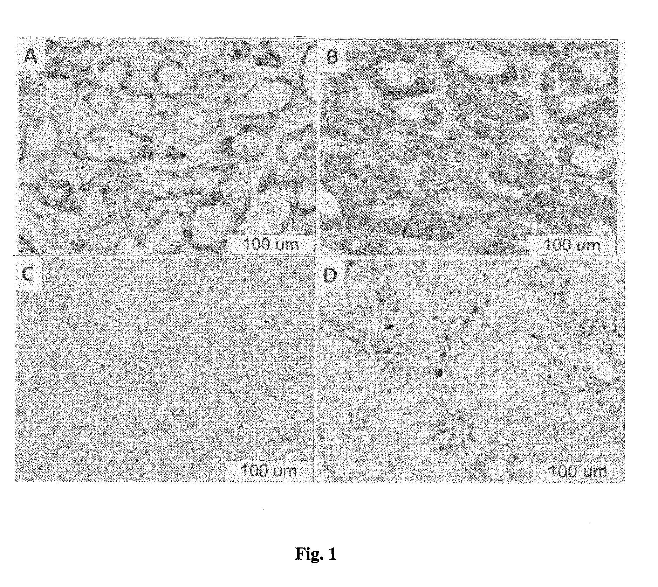

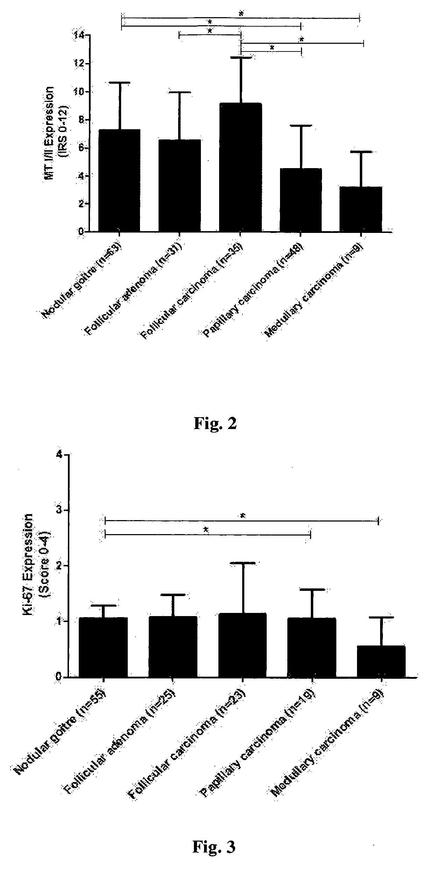

The subject of the present invention in the example embodiment is illustrated in the figures, wherein fig. 1 represents the nuclear-cytoplasmatic expression of MT in a follicular adenoma (1A) and follicular cancer (1B) and the expression of the nuclear antigen Ki-67 in follicular adenoma (1C) and follicular cancer (1D); fig. 2 represents the intensification of MT expression in various changes in the thyroid. A statistically significant difference was shown between follicular cancer and papillary cancer (p < 0.001) as well as follicular adenoma (p < 0.05). The statistically significant higher expression was also shown between follicular cancer and medullary cancer (Fig. 2; P < 0.0001). A statistically significant lower expression in relation to tumorous goitre was observed for papillary cancer (p < 0.0001) and medullar cancer (p < 0.005). Fig. 3 shows the intensification of the expression of antigen Ki-67 in various types of changes of the thyroid. A statistically significant difference was observed between medullar cancer and follicular adenoma (p < 0.05) and a medullar cancer and a tumorous goitre (p < 0.05).

The present invention is illustrated by the example given below.

The study made use of 186 paraffin blocks of various changes of the thyroid obtained from the Department of Pathomorphology of the Medical Academy of Wroclaw. The examined blocks contained 92 cancer cases, among these: 48 of papillary cancer, 35 of follicular cancer, 9 of medullar cancer, 31 of adenoma and 63 cases of tumorous goitre.

### A. Immunohistochemistry.

Tumour biopsies were fixed in 10% buffered formalin, dehydrated and then imbedded in paraffin blocks. All immunohistochemical (IHC) reactions were performed on paraffin sections 4µm thick, deparaffinated in xylene and rehydrated. For IHC anti-Ki-67 reactions, the sections were incubated in citrate buffer (pH 6.10 mM) at a temperature of 95-98°C for 20 minute in order to expose the epitopes. Endogenous peroxidase activity was blocked using a 5 minute incubation in 3% H₂0₂. The expression of MT I/II (clone E9) and Ki-67 (clone MIB-1) was demonstrated using murine monoclonal antibodies from DakoCytomation (Glostrup, Denmark). The examined antigens were visualised using biotinylated antibodies and streptavidin conjugated with horseradish peroxidase (DakoCytomation LSAB+System-HRP). The susbtrate used was diaminobenzidine (DAB, DakoCytomation). All reactions were performed using a negative control. All samples were counterstained with haematoxylin.

### B. Evaluation of the intensity of IHC reactions

To evaluate the intensity of MT expression we used a semiquantitative IRS method according to Remmele, in which we took into account the intensity of the chromogenic reaction and the percentage of positive oncogenic cells in a sample. This scale has a range from 0 to 12 points: 0 - no reaction, 1-2 - poor reaction, 3-4 - moderate reaction, 6-12 - strong reaction. The intensity of antigen Ki-67 in oncogenic cells was evaluated using a scale demonstrating the percentage of positive cells in relation to all oncogenic cells. This scale has the following values: 0 no reaction, 1 pt. - 1-10%, 2 pts. - 11-25%, 3 pts. - 26-50%, 4 pts. - >50%.

### C. Statistical analysis

The results were analysed statistically using the Prism 5.0 package (GraphPad, CA, USA). We used the Spearman correlation tests, the Kruskal-Wallis test and the Mann-Whitney U test. Statistically significant differences were deemed at a level of p<0.05

Among the 186 examined thyroid changes, in 180 (96,7%) we noted nuclear-cytoplasmatic MT expression (Fig. 1A, 1B). High MT expression (6-12 pkt) was seen in: 73% cases of tumorous goitre, 70.9% cases of follicular adenoma (Fig. 1A), 45.8% cases of papillary cancer and 85.7% cases of follicular cancer (Fig. 1B). High MT expression was noted in 33% cases of medullary cancer.

The highest MT expression was noted in follicular cancer (9.14±3.32), a lower but still significant level was noted in tumorous goitre (7.25±3.41), follicular adenoma (6.54±3.40), and the lowest in papillary cancer (4.47±3.14) and medullary cancer (3.22±2.54). Statistically significant differences were observed in MT expression between the individual thyroid changes (Fig. 2).

A statistically significant difference was observed between the intensity of MT expression in follicular cancer (Fig. 1B) and follicular adenoma (Fig. 1A), which are difficult to differentiate histologically (Fig. 2; P<0.05). A statistically significant higher expression was demonstrated in papillary cancer than in follicular cancer (Fig. 2; P< 0.0001) and medullary cancer (Fig. 2; P < 0.0001). A statistically significant lower expression in comparison to tumorous goitre was observed for papillary cancer (Fig. 2; P < 0.0001) and medullary cancer (Fig. 2; P < 0.005).

Among 131 examined changes, in 118 we discovered the nuclear expression of antigen Ki-67. The highest expression expression (Figure 3) was noted in follicular cancer (1.13±0.92) slightly lower in follicular adenoma (1.08±0.4), papillary cancer (1.05±0.52) and tumorous goitre (1.05±0.22) and the lowest in medullary cancer (0.56±0.53).

No significant difference were observed in the intensity of the expression of antigen Ki-67 between follicular adenoma (Fig. 1C) and follicular cancer (Fig. ID). A statistically significant difference in expression for this antigen was discovered between medullary cancer and tumorous goitre (p < 0.05), and between medullary cancer and follicular adenoma (p < 0.05) (Fig. 3).

The dependence of the expression metallothionein and antigen Ki-67 in changes such as follicular adenoma and follicular carcinoma evaluated using the Spearman test showed a lack of correlation between the abovementioned antigens (r = 0.12; p> 0.05).

## Claims

1. A method of differentiating follicular adenoma from follicular cancer of the thyroid gland comprising the following steps
(a) in a sample of oncogenic tissue from a patient the expression of isoforms of MT I and MT II of metallothionein protein is measured using immunochistochemical methods using monoclonal anti-MT antibodies,
(b) the degree of the expression of MT I and MT II is measured under a light microscope using Remmele IRS method,
wherein if the degree of intensity of the expression of metallothionein is equal to or greater than 8 on the IRS scale then the presence of follicular cancer is indicated.

2. A method according to Claim 1, **characterised in that** expression of MT I and MT II is examined in material taken from a patient.

3. A method according to Claims 1 or 2, **characterised in that** the presence of follicular cancer or follicular adenoma is determined along with a histological evaluation of said changes.

4. The use of metallothionein isoforms I or II for differentiating follicular adenoma from follicular cancer of the thyroid gland.

## Patentansprüche

1. Ein Verfahren zur Unterscheidung von follikulärem Adenom und Follikelkrebs der Schilddrüse, umfassend die folgenden Schritte
(a) in einer Probe von onkogenem Gewebe eines Patienten wird die Expression der Isoformen MT I und MT II von Metallothionein-Protein mittels immunhistochemischer Verfahren unter Verwendung monoklonaler anti-MT-Antikörper gemessen,
(b) der Grad der Expression von MT I und MT II wird unter einem Lichtmikroskop mittels des Remmele-IRS-Verfahrens gemessen,
wobei, falls der Intensitätsgrad der Expression von Metallothionein gleich oder größer als 8 auf der IRS-Skala ist, die Anwesenheit von Follikelkrebs angezeigt ist.

2. Ein Verfahren nach Anspruch 1, dadurch charakterisiert, dass die Expression von MT I und MT II in Material aus einem Patienten untersucht wird.

3. Ein Verfahren nach Ansprüchen 1 oder 2, dadurch charakterisiert, dass die Anwesenheit von Follikelkrebs oder Follikeladenom zusammen mit einer histologischen Evaluierung jener Änderungen bestimmt wird.

4. Die Verwendung von Metallothionein-Isoformen I oder II zur Unterscheidung von follikulärem Adenom und Follikelkrebs der Schilddrüse.

## Revendications

1. Un procédé de différenciation d'adénome folliculaire du cancer folliculaire de la glande thyroïde comprenant les étapes suivantes
(a) dans un échantillon de tissu oncogénique provenant d'un patient, l'expression des isoformes MT I et MT II de la protéine métallothionéine est mesurée au moyen de procédés d'immunohistochimie en utilisant des anticorps anti-MT monoclonaux,
(b) le degré d'expression de MT I et MT II est évalué au microscope optique au moyen de la méthode IRS selon Remmele,
selon lequel, si le degré d'intensité de l'expression de la métallothionéine est égal ou supérieur à 8 sur l'échelle d'IRS, la présence d'un cancer folliculaire est indiquée.

2. Le procédé selon la revendication 1, **caractérisée en ce que** l'expression de MT I et MT II est mesurée dans un échantillon prélevé sur un patient.

3. Le procédé selon la revendication 1 ou 2, **caractérisée en ce que** la présence d'un cancer folliculaire ou d'un adénome folliculaire est déterminée ainsi qu'une évaluation histologique de ces changements

4. Utilisation des isoformes I et II de la métallothionéine pour différencier d'adénome folliculaire du cancer folliculaire de la glande thyroïde.
